# EUROPÄISCHE PATENTSCHRIFT

(11) **EP 1 557 156 B1**
(45) Veröffentlichungstag und Bekanntmachung des Hinweises auf die Patenterteilung: **25.03.2009**
(21) Anmeldenummer: 04105541.9
(22) Anmeldetag: 05.11.2004
(51) Int. Cl.: A61K 8/44, A61Q 5/12

(54) **Kosmetische Zubereitung zur Pflege des Haares, enthaltend Lauroyl Lysin**
Cosmetic hair-care composition comprising lauroyl lysine
Composition cosmétique pour le soin des cheveux comprenant la lauroyl-lysine

(30) Priorität: 23.12.2003 DE 10361854
(43) Veröffentlichungstag der Anmeldung: 27.07.2005
(73) Patentinhaber: Beiersdorf AG, 20245 Hamburg (DE)
(72) Erfinder: Demitz, Michael, 22529, Hamburg (DE); Argembeaux, Horst, 21465, Wentorf (DE)

(56) Entgegenhaltungen:
- EP-A- 0 336 265
- EP-A- 1 384 470
- DE-A- 10 056 909
- DE-A- 19 916 209

## Beschreibung

Die vorliegende Erfindung betrifft Haarpflegeprodukte, insbesondere solche, die nach der Anwendung nicht ausgespült werden. Solche werden üblicherweise als "leave-on"- oder "stay-in"-Produkte bezeichnet, was andeutet, dass sie nach der Anwendung auf dem Haar oder in der Frisur verbleiben. Solche Zubereitungen tragen dazu bei, das Haar in seinem gesunden Zustand zu erhalten oder dieses in einen solchen Zustand zu versetzen. Solcherart behandeltes Haar zeichnet sich durch einen angenehmen Griff, natürlichen Glanz, Geschmeidigkeit und somit gute Frisierbarkeit und Festigkeit und somit gutem Frisurensitz aus. Produkte die ausschließlich der Pflege des Haares dienen werden allgemein als Conditioner bezeichnet.
Solche Zubereitungen enthalten üblicherweise kationische Tenside und kationische Polymere. Diese ziehen auf das Haar auf und führen zu einer Verbesserung der Kämmbarkeit und des Glanzes des Haares, wobei die Polymere gleichzeitig die Konsistenz der Zubereitungen verbessern.
Derartige Produkte können verschiedene Konsistenzen haben, so dass sie ganz unterschiedlich appliziert werden können. Es können Emulsionen oder Gele sein oder dünnflüssige Lösungen, die z.B. über Sprühapplikationen aufgebracht werden, oder Schäume, die z.B. durch geeignete Druckgaspackungen oder spezielle Schaumpumpen bei der Applikation erzeugt werden. Cremige, trübe und klar transparente Produkte sind im Markt zu finden.
Je nach Verwendungszweck findet man ganz unterschiedliche Wirkstoffe oder Kombinationen von Wirkstoffen in solchen Conditionern. Manche die eher dem Schutz des Haares dienen, wie Antioxidantien oder UV-Filter, andere die das Haar geschmeidig machen wie z.B. kationische Tenside. Eine immer größere Bedeutung bekommen polymere Wirkstoffe, die je nach Art, Molgewicht und Ladung ganz unterschiedliche Eigenschaften haben. Im Vordergrund steht dabei jedoch eindeutig eine Verbesserung der Oberflächenbeschaffenheit des Haares.
Trotz einer großen Vielfalt von kationischen Tensiden und Polymeren, die dem Fachman zur Verfügung stehen, sind einige Nachteile, die mit dem Einsatz dieser Stoffe, einzeln oder in Kombination, verbunden sind noch nicht restlos beseitigt. So zeigen kationische Tenside und / oder kationische Polymere häufig ein mangelghaftes sensorisches Verhalten auf dem Haar, was sich durch ein belegtes, häufig glitschiges, z. T. aber auch stumpfes, manchmal etwas klebriges Griffempfinden bemerkbar macht. Dieses Verhalten tritt besonders bei leave-on Produkten auf.
Eine Aufgabe dieser Erfindung war es, eine Zubereitung zur Haarpflege bereitzustellen, die dieses mangelhafte sensorische Verhalten nicht aufweist.
Lauroyl Lysine stellt das Additionsprodukt N(epsilon)-Lauroyl-L-Lysin aus L-Lysin und Laurinsäure dar. Es wird durch folgende Formel beschrieben:

C₁₁H₂₃-CO-NH-(CH₂)₄-CH(-NH₃⁺)-COO⁻

Es kann in Form eines Pulvers als Pudergrundlage verwendet werden. Aufgrund seiner partikulären Blättchenstruktur weisen die Teilchen auf der Haut ein einzigartiges Gleitverhalten auf. Dabei fühlern sich die 10-20µm messenden Teilchen weich an und quellen in Wasser oder Ölen nicht auf. Eine solche Pudergrundlage wird beispielsweise von der Fa. Ajinomoto unter der Bezeichnung Amihope LL angeboten.

Es hat sich für den Fachmann nicht vorhersehbar herausgestellt, dass eine Zubereitung zur Haarpflege enthaltend Lauroyl Lysine, besonders bevorzugt Amihope LL von der Fa. Ajinomoto angeboten, kationische Tensiden den Mängeln des Standes der Technik abhelfen.
Dadurch werden in haarpflegenden Conditionern hervorragende Pflegeleistungen erhalten. Die Kombination von Aluminiumsalzen kationischer Stärkederivate mit kationischen Tensiden und/oder Polymeren verbessert synergistisch die geschmeidigkeitsverbessernden Pflegeeigenschaften ohne das Haar zu beschweren. Gleichfalls erhält das Haar ein angenehm weiches, unbeschwertes Griffempfinden und läßt sich gut frisieren. Die erfindungsgemäßen Formulierungen zeichnen sich darüber hinaus noch durch eine angenehme weiche, gut verteilbare Textur aus.
Besonders vorteilhaft ist der Einsatz einer Kombination von Aluminium Starch Octenylsuccinaten mit Homo- oder Copolymere von Ester- oder Amidderivaten der Acryl- oder Methacrylsäure, besonders Homopolymere aus Methacryloylethyltrimethylammonium Chlorid (INCI: Polyquaternium-37)(Syntalen CR von 3 V). Ganz besonders vorteilhaft ist der Einsatz dieser Kombination mit weitern kationischen Polymeren, besonders Quaternären Copolymeren aus Hydroxyethylcellulose und Diallyl-demethylammoniumchlorid (INCI: Polyquaternium-4) (Celquat L-200 von National Starch)). Weiter bevorzugt ist es, wenn die Einsatzkonzentrationen von Lauroyl Lysin im Bereich von 0,01 bis 5% liegen, bezogen auf das Gesamtgewicht solcher Zubereitungen.
Die Erfindung umfasst auch eine solche Zubereitung, die nach der Anwendung auf dem Haar verbleibt sowie eine solche Zubereitung, die als Gel, Emulsion, Spray, schaumförmige oder aufschäumbare Zubereitung in einem geeigneten Packmittel vorliegt, im Fall schaumförmiger oder aufschäumbarer Zubereitungen in einem Schaumspender oder einer Schaumpumpe.
Erfindungsgemäß ist auch die Verwendung von solchen Zubereitungen zur Haarpflege, wobei sie nach der Anwendung auf dem Haar mindestens 30 Minuten verbleiben sowie ein kosmetisches Behandlungsverfahren umfassend die Schritte (a) Anfeuchten der Haare, (b) Auftragen und Einmassieren einer beschriebenen Zubereitung, (c) Trocknen und gegebenenfalls Frisieren des Haares, wobei auf ein Ausspülen der Zubereitung verzichtet wird.
Weiter betrifft die Erfindung auch ein Produkt umfassend ein beschriftetes Packmittel und eine darin enthaltene oben beschriebene Zubereitung wobei die Beschriftung auf die genannte Verwendung oder das genannte Verfahren hinweist.

Das Weglassen eines einzelnen Bestandteile beeinträchtigt die einzigartigen Eigenschaften der Gesamtzusammensetzung. Daher sind alle angegebenen Bestandteile der erfindungsgemäßen Zubereitungen zwangsläufig erforderlich, um die Erfindung auszuführen.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-ÖI (W/O) oder vom Typ ÖI-in-Wasser (O/W), eine multiple Emulsionen, beispielsweise vom Typ Wasser-in-ÖI-in-Wasser (W/O/W), ein Gel, einen festen Stift, eine Salbe oder auch ein Aerosol darstellen.

Die Haarpflegemittel sind topische Zubereitungen. Diese können wie üblich zusammengesetzt sein und zur Behandlung und der Pflege der Kopfhaut und/oder der Haare oder als Lichtschutzpräparat dienen. Zur Anwendung werden die erfindungsgemäßen Zubereitungen in der für Kosmetika und Haarpflegemittel üblichen Weise auf die Kopfhaut und die Haare in ausreichender Menge aufgebracht.

Vorteilhaft können Zubereitungen im Sinne der vorliegenden Erfindung als Haarkur oder Haarspülung vorliegen.

Die Mittel gemäß der Erfindung können beispielsweise als aus Aerosolbehältern, Quetschflaschen oder durch eine Pump-, Sprüh- oder Schaumvorrichtung versprühbare Präparate vorliegen, jedoch auch in Form eines aus normalen Flaschen und Behältern auftragbaren Mittels.

Als Treibmittel für aus Aerosolbehältern versprühbare kosmetische oder dermatologische Zubereitungen im Sinne der vorliegenden Erfindung, sind die üblichen bekannten leichtflüchtigen, verflüssigten Treibmittel, beispielsweise Dimethylether, Kohlenwasserstoffe (Propan, Butan, Isobutan) geeignet, die allein oder in Mischung miteinander eingesetzt werden können. Auch Druckluft, Stickstoff, Stickstoffdioxid oder Kohlendioxid oder Gemische aus diesen Substanzen sind vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Vorteilhaft enthalten erfindungsgemäße Zubereitungen neben einem wirksamen Gehalt an erfindungsgemäßen Wirkstoffen ferner übliche Wirk-, Inhalts-, Zusatz- und/oder Hilfsstoffe.

Als Haarpflegemittel wird eine Vielzahl von Produkten bezeichnet, deren wichtigste Vertreter Vorbehandlungsmittel, Haarwässer und Haarkonditioniermittel sind.

Grundstoffe für Haarpflegemittel sind z.B. Fettalkohole, Wachse, Paraffine, Vaseline, Paraffinöl und Lösemittel.

Fettalkohole sind z.B. gerad- oder verzweigtkettige aliphatische einwertige Alkohole mit 6 - 22 C-Atomen im Molekül. In der Kosmetik werden vorzugsweise geradkettige Fettalkohole mit einer Kettenlänge von 12 - 18 C-Atomen verwendet. Diese Fettalkohole sind weiche, farblose Massen, praktisch ungiftig und gut hautverträglich. Fettalkohole werden bevorzugt zur Herstellung von Haarkuren und Frisiercremes verwendet, wobei dem Cetylalkohol und dem Stearylalkohol besondere Bedeutung zukommt.

Wachse sind Fettsäureester, die in tierischen und pflanzlichen Produkten vorkommen, aber auch synthetisch hergestellt werden können. Das wohl bekannteste natürlich vorkommende Wachs ist das Bienenwachs, das als Hauptbestandteile Cerin und Myricin enthält. Wachs ist jedoch ein Oberbegriff für eine Reihe natürlich oder künstlich gewonnener Stoffe, die in der Regel halbfeste, weiße, geruchlose und in Wasser unlösliche Massen darstellen.

Paraffine im kosmetischen Sinn sind weiße, geruchlose Massen aus geradkettigen hochmolekularen Kohlenwasserstoffen. Wegen ihrer den der Wachse vergleichbaren Eigenschaften werden sie auch oft als Erdölwachse bezeichnet.

Vaseline ist ein Gemisch von verzweigtkettigen Paraffinen mit geringem Anteil an zyklischen Paraffinen. Es ist eine weiche, transparente und in Wasser unlösliche Masse mit geringem Eigengeruch, die bei der Aufbereitung des Erdöls anfällt.

Paraffinöl ist ein Gemisch gesättigter flüssiger Kohlenwasserstoffe. Es ist unlöslich in Wasser, aber mischbar mit Fettalkoholen und Wachsen. Es wird als Zusatz in Haarpflegemitteln zur Konsistenzregulierung verwendet.

Lösemittel spielen in der Kosmetik eine erhebliche Rolle. Von der großen Anzahl Lösemittel, die zur Verfügung stehen, hat Ethanol die größte Bedeutung. Es wird zur Herstellung von Haarwässern verwendet, in denen es wegen seiner desinfizierenden Eigenschaften gleichzeitig die Funktion eines Wirkstoffes erfüllt. Gelegentlich wird auch Isopropanol für diese Zwecke eingesetzt.

Hilfstoffe können verwendet werden, um bestimmte Eigenschaften der Haarpflegemittel, z.B. Konsistenz, Temperatur- und Lichtstabilität, Aussehen und Geruch, zu verbessern sowie deren Herstellung zu erleichtern. Zugesetzt werden z.B. nach Bedarf:
- Emulgatoren, um die Grenzflächenspannung zwischen zwei an sich nicht mischbaren Phasen so weit herabzusetzen, daß deren feine Vermischung möglich wird,
- Verdickungsmittel, um die Stabilität von Emulsionen zu erhöhen und deren Viskosität einzustellen,
- UV-Absorber, um die Lichtstabilität der in Haarpflegemitteln enthaltenen Farbstoffe und anderer lichtempfindlicher Komponenten zu verbessern. Daneben dienen sie dem Schutz des Haares vor Lichteinflüssen.
- Konservierungsmittel, um mikrobieller Zersetzung vorzubeugen.
- Antioxidantien, um Geruchsveränderungen, die durch Oxidationsvorgänge hervorgerufen werden können, zu verhindern.
- Farbstoffe, um Haarpflegemitteln ein ansprechendes Aussehen zu verleihen.
- Parfümöle, um Haarpflegemitteln einen angenehmen Duft zu verleihen und Nebengerüche der Rohstoffe zu überdecken.

Die Menge der Grundstoffe beträgt beispielsweise 85 bis 99,999 Gew.-%, vorzugsweise 90 bis 99,99 Gew.-%, jeweils bezogen auf das Gesamtgewicht der Zubereitung.

Quaternäre Ammonium-Verbindungen sind eine wichtige Gruppe der speziellen Wirkstoffe, die zur Herstellung von Haarpflegemitteln verwendet werden. Haarpflegemittel, insbesondere Haarkuren, erhalten wesentliche Eigenschaften wie Verbesserung von Kämmbarkeit sowie Griff und Verhinderung statischer Aufladung der Haare vornehmlich durch den Einsatz quaternärer Ammonium-Verbindungen.
Die Eigenschaften quaternärer Ammonium-Verbindungen werden durch die kationische Gruppe einerseits und durch die Art der lipophilen Reste dieser Gruppe andererseits bestimmt. Geeignet sind z.B. die Verbindungen, in denen ein bis zwei Reste längerkettige Alkyl-Gruppen, wie Lauryl-, Cetyl- oder Stearyl-Gruppen, und die verbliebenen Reste Methyl-Gruppen sind. Produkte dieses Typs werden vorzugsweise als Chloride, Bromide und Methosulfate eingesetzt.

Geeignet sind auch polymere quaternäre Ammonium-Verbindungen, Makromoleküle, deren wesentliches Merkmal das Vorhandensein mehrerer quaternärer Ammonium-Gruppen im Molekül ist. Dadurch wird ihre Haftfähigkeit am Haar deutlich erhöht.

Besonders vorteilhaft zu verwendende kationische Tenside sind
1. Alkylamine
2. Alkylimidazole
3. Ethoxylierte Amine und
4. Quaternäre Tenside.
5. Esterquats

Quaternäre Tenside erhalten mindestens ein N-Atom, das mit 4 Alkyl- oder Arylgruppen kovalent verbunden ist. Dies führt, unabhängig vom pH-Wert, zu einer positiven Ladung. Vorteilhaft sind Alkylbetain, Alkylamidopropylbetain und Alkyl-amidopropylhydroxysultain. Die erfindungsgemäß verwendeten kationischen Tenside können ferner bevorzugt gewählt werden aus der Gruppe der quaternären Ammoniumverbindungen, insbesondere Benzyltrialkylammoniumchlorid, ferner Alkyltrialkylammoniumsalze, beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Monomere oder polymere quaternäre Ammonium-Verbindungen werden vielfach in Haarspülungen und Haarkuren z.B. in Konzentrationen von 0,5 - 5 Gew.-% eingesetzt. Dazu gehören Cetrimoniumchlorid wie es unter der Bezeichnung Dehyquart A von der Gesellschaft Cognis angeboten wird oder Distearoylethyl Hydroxyethylmonium Methosulfate wie es unter der Bezeichnung Dehyquart F 75 von der Gesellschaft Cognis angeboten wird.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um Emulsionen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine Emulsion dar und enthält die erfindungsgemäßen Kombinationen. Es ist allerdings gegebenenfalls vorteilhaft, wenn die erfindungsgemäße Lotion in Form einer Mikroemulsion oder einer wäßrigen oder wäßrigalkoholischen Lösung vorliegt.

Erfindungsgemäß können kosmetische Zubereitungen zur Behandlung und Pflege der Haare als Gele vorliegen, die organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Die vorstehenden Prozentangaben beziehen sich auf das Gesamtgewicht der Zubereitungen.

Die erfindungsgemäßen Zubereitungen können kosmetische Hilfsstoffe enthalten, wie sie üblicherweise in solchen Zubereitungen verwendet werden, z.B. Konservierungsmittel, Parfüme, Substanzen zum Verhindern des Schäumens, Schaumstabilisatoren, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, rückfettende Agentien, Fette, Öle, Wachse, Alkohole, Polyole und deren toxikologisch verträglichen Ether und Ester, verzweigte und/oder unverzweigte Kohlenwasserstoffe, weitere Antioxidantien, Stabilisatoren, pH-Wert-Regulatoren, Konsistenzgeber, Bakterizide, Desodorantien, antimikrobielle Stoffe, Antistatika, UV-Absorber, Komplexierungs- und Sequestrierungsagentien, Perlglanzagentien, Polymere, Elektrolyte, organische Lösungsmittel, Silikonderivate, Pflanzenextrakte, Vitamine und/oder andere Wirkstoffe oder andere übliche Bestandteile einer kosmetischen oder dermatologischen Formulierung. Auch Lösungsvermittler, z.B. zur Einarbeitung hydrophober Komponenten wie z.B. von Parfümzubereitungen können enthalten sein.

Die Gesamtmenge der Hilfsstoffe beträgt beispielsweise 0,001 bis 15 Gew.-%, vorzugsweise 0,01 bis 10 Gew.-%, jeweils bezogen auf das Gesamtgewicht der Zubereitung.

Die Menge der Verdickungsmittel beträgt beispielsweise 0,05 bis 5,0 Gew.-%, vorzugsweise 0,1 bis 3,0 Gew.-%, insbesondere 0,15 bis 2,0 Gew.-%, jeweils bezogen auf das Gesamtgewicht der Zubereitung.

Der Wassergehalt der Zubereitungen beträgt beispielsweise 60 bis 95 Gew.-%, vorzugsweise 75 bis 95 Gew.-%, insbesondere 80 bis 90 Gew.-%, jeweils bezogen auf das Gesamtgewicht der Zubereitung.

Erfindungsgemäß können zusätzlich als Antioxidantien alle für kosmetische und/oder dermatologische Anwendungen geeigneten oder gebräuchlichen Antioxidantien verwendet werden.

Die Gesamtmenge der Antioxidantien beträgt beispielsweise 0,000.001 bis 2 Gew.-%, vorzugsweise 0,001 bis 1 Gew.-%, jeweils bezogen auf das Gesamtgewicht der Zubereitung.

Vorteilhaft werden weitere Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Vorteilhaft können erfindungsgemäße Zubereitungen außerdem Substanzen enthalten, die UV-Strahlung im UV-B-Bereich absorbieren, wobei die Gesamtmenge der Filtersubstanzen z.B. 0,001 Gew.-% bis 30 Gew.-%, vorzugsweise 0,05 bis 10 Gew.-%, insbesondere 0,1 bis 1,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut, insbesondere die Kopfhaut dienen.

Enthalten die erfindungsgemäßen Emulsionen UV-B-Filtersubstanzen, können diese vorteilhaft wasserlöslich sein. Vorteilhafte wasserlösliche UV-B-Filter sind z.B.:
- Salze der 2-Phenylbenzimidazol-5-sulfonsäure wie ihr Natrium-, Kalium- oder ihr Triethanolammonium-Salz, sowie die 2-Phenylbenzimidazol-5-sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Es kann auch von Vorteil sein, erfindungsgemäße Zubereitungen mit UV-A-Filtern zu versetzen, die bisher üblicherweise in kosmetischen Zubereitungen enthalten sind. Es können die für die UV-B-Kombination verwendeten Mengen eingesetzt werden.

Die Herstellung der erfindungsgemäßen Zubereitungen kann in der üblichen Weise durch Mischen der einzelnen Bestandteile erfolgen. Die Wirkstoffe der erfindungsgemäßen Kombinationen oder auch die vorgemischten Bestandteile der erfindungsgemäßen Kombinationen können im Mischvorgang zugegeben werden.

Der pH-Wert der Zubereitungen kann in bekannter Weise durch Zugabe von Säuren oder Basen eingestellt werden, vorzugsweise durch Zugabe von Puffergemischen, z.B. auf Basis von Citronensäure/Citrat oder Phosphorsäure Phosphat-Puffergemischen. Vorzugsweise liegt der pH-Wert unter 10, z.B. im Bereich von 2-7, insbesondere im Bereich von 3-5.

Alle Mengenangaben, Anteile und Prozentanteile sind, soweit nicht anders angegeben, auf das Gewicht und die Gesamtmenge bzw. auf das Gesamtgewicht der Zubereitungen oder der jeweiligen Mischung bezogen.
Es ist bei all diesem im Einzelfalle möglich, daß die vorgenannten Konzentrationsangaben leicht über- oder unterschritten werden und dennoch erfindungsgemäße Zubereitungen erhalten werden. Dies kommt angesichts der breit streuenden Vielfalt an geeigneten Komponenten derartiger Zubereitungen für den Fachmann nicht unerwartet, so daß er weiß, daß bei solchen Überoder Unterschreitungen der Boden der vorliegenden Erfindung nicht verlassen wird.
Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele

### Beispiele 1-3 Haarkuren

| | **1** | **2** | **3** |
|---|---|---|---|
| Polyquaternium-37 | 0,5 | 0,5 | 0,5 |
| Cetrimoniumbromid | 1,0 | 1,0 | 1,0 |
| Glycerin | 3,0 | 3,0 | 3,0 |
| Cetearylalkohol | 2,5 | 2,5 | 2,5 |
| Glycerylstearat | 2,0 | 2,0 | 2,0 |
| Lauroyl Lysine | 0,2 | 1,0 | 0,8 |
| Polyquaternium-10 | 0,1 | - | - |
| | | | |
| Konservierungsmittel, Parfüm, pH-Einstellung | q.s. | q.s. | q.s. |
| Wasser, VES (vollentsalzt) | ad 100,0 | ad 100,0 | ad 100,0 |

Der pH-Wert wird auf 3,5 eingestellt.

### Beispiele 4 - 6 Haarspülungen

| | **4** | **5** | **6** |
|---|---|---|---|
| Behentrimoniumchlorid | 1,0 | 1,0 | 1,0 |
| Glycerin | 3,0 | 3,0 | 3,0 |
| Polyquaternium-37 | 0,2 | 0,2 | 0,2 |
| Cetearylalkohol | 3,0 | 3,0 | 3,0 |
| Lauroyl Lysine | 0,2 | 0,5 | 0,8 |
| Polyquaternium-10 | 0,1 | - | - |
| Guar Hydroxypropyl Trimonium Chlorid | - | 0,2 | - |
| Konservierungsmittel, Parfüm, pH-Einstellung | q.s. | q.s. | q.s. |
| Wasser, VES | ad 100,0 | ad 100,0 | ad 100,0 |
| Der pH-Wert wird auf 3,0 eingestellt. | | | |

### Beispiele 7 - 9 Leave-on Conditioner

| | **10** | **11** | **12** |
|---|---|---|---|
| Cetylalcohol | - | 1,8 | 2,0 |
| C12-13 Alkyl Lactate | 2,0 | 1,0 | 1,5 |
| Cetrimonium chlorid | 0,3 | 0,2 | 0,2 |
| Polyquaternium-37 | 1,0 | 0,3 | 0,2 |
| Polyquaternium-4 | 0,2 | 0,2 | 0,1 |
| Lauroyl Lysine | 1,0 | 0,8 | 1,2 |
| Konservierungsmittel, Parfüm, pH-Einstellung | q.s. | q.s. | q.s. |
| Wasser, VES | ad 100,0 | ad 100,0 | ad 100,0 |

## Patentansprüche

1. Zubereitung zur Haarpflege enthaltend
Lauroyl Lysin und
kationische Tenside und als kationisches Polymer. eine Kombination von Aluminium Starch Octenylsuccinaten mit Homo- oder Copolymeren - von Ester- oder Amidderivaten der Acryl- oder Methacrylsäure.

2. Zubereitung nach einem der vorangehenden Ansprüche **dadurch gekennzeichnet, dass** die Einsatzkonzentrationen von Lauroyl Lysin im Bereich von 0,01 bis 5% liegen, bezogen auf das Gesamtgewicht solcher Zubereitungen.

3. Zubereitung nach einem der vorangehenden Ansprüche **dadurch gekennzeichnet, daß** sie nach der Anwendung auf dem Haar verbleiben.

4. Zubereitung nach einem der vorangehenden Ansprüche **dadurch gekennzeichnet, daß** sie als Gel, Emulsion, Spray, schaumförmige oder aufschäumbare Zubereitung in einem geeigneten Packmittel vorliegt, im Fall schaumförmiger oder aufschäumbarer Zuberitungen in einem Schaumspender oder einer Schaumpumpe.

5. Verwendung von Zubereitungen nach einem der vorangehenden Ansprüche **dadurch gekennzeichnet, daß** sie nach der Anwendung auf dem Haar mindestens 30 Minuten verbleiben.

6. Kosmetisches Behandlunngsverfahren umfassend die Schritte
(a) Anfeuchten der Haare,
(b) Auftragen und Einmassieren einer Zubereitung nach einem der vorangehenden Ansprüche,
(c) Trocknen und gegebenenfalls Frisieren des Haares,
wobei auf ein Ausspülen der Zubereitung verzichtet wird.

7. Produkt umfassend ein beschriftetes Packmittel und eine darin enthaltene Zubereitung nach einem der vorangehenden Ansprüche wobei die Beschriftung auf eine Verwendung nach Anspruch 7 oder ein Verfahren nach Anspruch 8 hinweist.

## Claims

1. Hair-care composition comprising
lauroyl lysine and
cationic surfactants and, as cationic polymer, a combination of aluminium starch octenylsuccinates with homo- or copolymers of ester or amide derivatives of acrylic acid or methacrylic acid.

2. Composition according to one of the preceding claims, **characterized in that** the use concentrations of lauroyl lysine are in the range from 0.01 to 5%, based on the total weight of such compositions.

3. Composition according to one of the preceding claims, **characterized in that** they remain on the hair following application.

4. Composition according to one of the preceding claims, **characterized in that** it is in the form of a gel, emulsion, spray, foam-like or foamable composition in a suitable packaging, in the case of foam-like or foamable compositions in a foam dispenser or a foam pump.

5. Use of compositions according to one of the preceding claims, **characterized in that** they remain on the hair for at least 30 minutes following application.

6. Cosmetic treatment method comprising the steps
(a) wetting the hair,
(b) applying and massaging in a composition according to one of the preceding claims,
(c) drying and optionally styling the hair,
with a rinsing out of the composition being dispensed with.

7. Product comprising a labelled packaging and a composition according to one of the preceding claims contained therein, with the labelling referring to a use according to claim 5 or a method according to claim 6.

## Revendications

1. Préparation destinée au soin des cheveux, contenant
de la lauroyl-lysine et
des tensioactifs anioniques et en tant que polymère cationique une association d'amidon-octénylsuccinates d'aluminium avec des homo- ou copolymères de dérivés ester ou amide de l'acide acrylique ou méthacrylique.

2. Préparation selon l'une quelconque des revendications précédentes, **caractérisée en ce que** les concentrations utilisées de lauroyl-lysine se situent dans la plage de 0,01 à 5 % en poids, par rapport au poids total de telles préparations.

3. Préparation selon l'une quelconque des revendications précédentes, **caractérisée en ce qu'**elles restent sur le cheveu après l'emploi.

4. Préparation selon l'une quelconque des revendications précédentes, **caractérisée en ce qu'**elle se trouve sous forme de gel, d'émulsion, de préparation à pulvériser en aérosol, de préparation sous forme de mousse ou pouvant être transformée en mousse dans un emballage approprié, dans le cas de préparations sous forme de mousse ou pouvant être transformées en mousse, dans un distributeur de mousse ou un distributeur de mousse à pompe.

5. Utilisation de préparations selon l'une quelconque des revendications précédentes, **caractérisée en ce qu'**après l'emploi elles restent au moins 30 minutes sur le cheveu.

6. Procédé de traitement cosmétique, comprenant les étapes consistant à
(a) mouiller les cheveux,
(b) appliquer et faire pénétrer en massant une préparation selon l'une quelconque des revendications précédentes,
(c) sécher et éventuellement coiffer les cheveux,
sans éliminer par rinçage la préparation.

7. Produit comprenant un emballage portant une inscription et une préparation selon l'une quelconque des revendications précédentes, contenue dans celui-ci, l'inscription indiquant une utilisation selon la revendication 5 ou un procédé selon la revendication 6.
